# EUROPEAN PATENT SPECIFICATION

(11) **EP 1 578 978 B1**
(45) Date of publication and mention of the grant of the patent: **29.11.2006**
(21) Application number: 02788358.6
(22) Date of filing: 16.12.2002
(51) Int. Cl.: C12P 13/00, C12P 13/22

(54) **PROCESS FOR PREPARATION OF DOPA AND DOPAMINE FROM HAIRY ROOT CULTURES OF BETA VULGARIS**
VERFAHREN ZUR HERSTELLUNG VON DOPA UND DOPAMIN AUS HAARWURZELKULTUREN VON BETA VULGARIS
PROCEDE DE PREPARATION DE DOPA ET DE DOPAMINE A PARTIR DE CULTURES A RACINE CHEVELUE DE BETA VULGARIS

(43) Date of publication of application: 28.09.2005
(73) Proprietor: COUNCIL OF SCIENTIFIC AND INDUSTRIAL RESEARCH, New Delhi 110 001 (IN)
(72) Inventor: GOKARE, Ravishankar A. Central Food Tech Res Inst., Karnataka (IN); BHAMIDI, Suresh Central Food Techn. Res. Inst., Karnataka (IN); RAO, Ramachandra S. Central Food Techn. Res. Inst., Karnaka (IN)
(74) Representative: Jorio, Paolo
(86) International application number: PCT/IB2002/005390
(87) International publication number: WO 2004/055192

(56) References cited:
- EP-A- 0 189 938
- KOBAYASHI N ET AL: "Formation and occurrence of dopamine-derived betacyanins" PHYTOCHEMISTRY, PERGAMON PRESS, GB, vol. 56, no. 5, March 2001 (2001-03), pages 429-436, XP004291787 ISSN: 0031-9422

## Description

### Field of the invention

The present invention relates to a process for producing Dopa and dopamine from hairy root cultures of *Beta vulgaris.*

### Background and Prior art references

L-Dopa (Dihydroxy L-phenylalanine) and dopamine are neurotransmittor presursors being used for sympotomic relief of parkinson's disease (Pras, N et al 1989, Biotechnology and Bioengineering 34, 214-222). The high price, demand and high dose led for alternative and inexpensive methods for their production. L-Dopa is mainly produced as a secondary metabolite in plant cell cultures of *Mucuna pruriens* (Pras et al 1989) and callus as well as transformed root cultures of *Stizolobium hassjoo* (Sung and Huang 2000. Biotech Prog, 16, 1135-1140). L-Dopa is also produced by various bacteria viz. *Escherichia coli* and *Erwinia herbicola* (Foor et al 1993, Appl. Environ. Microbilo 59: 3070-3075) and *Psudomonas melanogenum* (Tanaka et al 1974, Agri Biol Chem, 38(3), 633-639).

Reference may be made to Wichers et al (1993) (PCTOC 33, 259-264), where both Dopa and dopamine were detected in plant organs such as leaves as well as cell cultures of *Mucuna pruriens.* They have also reported that addition of 2,4-D to cell suspensions increased Dopamine content and addition of NaCl at a concentration of 0.5 M helped in release of L-Dopa into the medium.

Various biotransformation processes were also reported for the production of L-Dopa viz. Bioconversion of tyrosine to L-Dopa by immobilized cells of *Mucuna pruriens* (Wichers et al 1983, Planta 158: 482-486), and also by immobilized cells of *Papaver somniferum* ( Stano et al 1995, phytochemistry, 38(4) 859-860).

*Beta vulgaris* hairy root cultures are known to produce betalaines, which are natural red pigments. Due to conjugated double bonds, they are colored. Among higher plants, the occurrence of betalaines is restricted to order centrospermae. However, they are also reported in mushroom *Amanita muscaria.* Interestingly, in microorganisms as well as in animals betalaines are unknown. (Bohm and Rink 1988).Betalaines, the yellow betaxanthins and red-violet betacyanins are a group of water soluble pigments. The betaxanthins are conjugates of betalamic acid with amino acids, are corresponding amines (including Dopamine), and the betacyanins are derivatives of betanidin, the conjugate of betalamic acid with cyclo-Dopa. Tyrosinase and Dopa dioxygenase are the main enzymes responsible for biosynthesis of basic skeleton of betalaines. (Mueller et al 1996, Steiner et al 1996, 1999, Girod and Zryd 1991, Mueller et al 1997). Tyrosinase is responsible for the formation of DOPA and its oxidation to cyclo-Dopa, the Dopa dioxygenase catalyses the Dopa extradiol cleavage, leading to the formation of betalamic acid. The decisive step, the linkage of betalamic acid with cyclo-Dopa was suggested to be a non enzymatic step( Trezzini and Zyrd 1990, Terradas and Wyler 1991). Kobayashi et al (2001) reported the formation of betacyanins from Dopamine in cell and hairy root cultures of *Beta vulgaris.*

Reference may be made to Steiner et al (Planta 1999, 208: 114-124) where they have found that tyrosinase is involved in betalaine biosynthesis of higher plants taking callus cultures of *Portulaca grandiflora* which produces betacyanins. They have also reported that betalaines in hairy roots and callus of *Beta vulgaris* are formed by the action of tyrosinase. Schliemann et al 1998 (Phytochemistry 49(6) 1593-1598) reported that betalaine formation from L-Dopa in a model assay system. In their experiments, they have incubated Dopa with Dopa dioxygenase from *Amanita muscaria,* which resulted in formation of 4,5-Seco-Dopa that spontaneously, recycled to betalamic acid.

In all the reports cited above, it was said that in biosynthetic pathway of betalaines, Dopa is a precursor of betalaines but till date there is no report on the accumulation / production of Dopa and dopamine in hairy root cultures of *Beta vulgaris.*

### Objects of the invention

The main object of the present invention is to develop a process for extraction and production of Dopa and Dopamine from *Beta vulgaris* hairy roots.

Another object of the invention is to provide a procedure for enhancement of its production of Dopa and Dopamine from *Beta vulgaris* hairy roots

### Summary of the Invention

Accordingly, the present invention provides a process for the production of Dopa and dopamine from hairy roots of *Beta vulgaris,* where the hairy roots were obtained upon infection of the explant using an isolate *of Agrobacterium rhizogenes* wild strain and were maintained by sub-culturing in MS (Murashige-Skoog 1962, Physiol plant 15, 473-479), medium without any phytohormones and with the addition of 3% sucrose and the pH of the medium was adjusted to 5.8 before autoclaving and the flasks were kept on a shaker at 90 rpm and cultured in dark for 24 days of culture time where in the hairy roots at different staged of growth were extracted with 2% formic acid, 0.1 N HCl and 80% hot ethanol in a mortar and pestle using glass powder, filtered and the extract was concentrated under vacuum and the concentrate was filtered through a membrane filter (0.22µm) and injected for HPLC analysis and the Dopa and dopamine contents were quantified by comparison of areas with authentic standards (Sigma, St.Louis USA) and experiments were carried out to know which stage of the culture accumulated maximum Dopa and dopamine contents which include the addition of tyrosine at exponential stage of culture time and found that hairy root cultures in the initial stages accumulated more Dopa (6-day old cultures) and at 15-days, the dopamine accumulation was maximum and when tyrosine was (Hi media labs, Mumbai, India) supplemented at 3mg/40ml culture medium, the Dopa content was maximum i.e. 46 µg/g DW on 15^{th} day and the dopamine content was also increased to 1.972 mg/g DW on 20^{th} day of culture time.

### Detailed Description of the Invention

Accordingly, the present invention provides a process for the production of Dopa and Dopamine from hairy root culture of *B. vulgaris,* said process comprising steps of:
a) culturing of hairy roots of *B. vulgaris* using nutrient media, sterilizing in an autoclave;
b) inoculating hairy roots in the range of 0.1-0.5 g fresh wt/40ml culture medium, grown for 2-24 days to obtain higher growth and metabolites;
c) choosing the right stage of culture of hairy roots for maximum Dopa and Dopamine production based on the biosynthetic pathway of betalaines; and
d) extracting the hairy roots with solvent to obtain Dopa and dopamine.

In an embodiment of the invention, the nutrient media used comprising MS medium with 2 to 4 % of sucrose as a major carbon source.

Still another embodiment of the invention provides a process wherein, sterilization in step (a) is carried out for 10 to 30 minutes at a temperature in the range of 110 to 130°C.

Still another embodiment, wherein in step (d), the hairy roots are extracted using formic acid in the range of 1-4 % (v/v), 0.1 N HCl and 80% hot ethanol.

Yet another embodiment, the extracts are centrifuged, filtered and concentrated under vacuum.

Yet another embodiment, addition of formic acid in the range of 1-4 % (V/V) for extraction enhances the production of Dopa and dopamine.

In yet another embodiment, further addition of tyrosine in the range of 1-5 mg/40-ml culture medium to the culture of 3- 7 days old, resulting in enhanced yield of Dopa and dopamine.

Yet another emboidment, the hairy roots of beta vulgaris are cultured in nutrient liquid medium aseptically and extracted with 1-5 % formic acid at exponential growth.

Yet another embodiment, the exponential growth phase in step (c) for maximum accumulation of Dopa in culture ranges between 5- 8 days.

Yet another embodiment, in step (c), the exponential growth phase for maximum accumulation of Dopamine in culture ranges between 12- 16 days.

In an embodiment of the present invention, hairy roots of red beet-root were obtained using the wild strain of *Agrobacterium rhizogenes.*

In yet another embodiment of present invention, they were cultured on Murashige and Skoog's basal medium in 150-ml Erlenmeyer flasks.

In yet another embodiment of present invention, hairy roots of *B. vulgaris* were extracted at different growth stages with 2% (v/v) formic acid in water, 0.1N HCl and 80% hot ethanol.

In yet another embodiment of present invention, the extracts were centrifuged, filtered through a whatman#1 filter paper and concentrated under vacuum, dissolved in respective solvents such as 2% (v/v) formic acid in water, 0.1N HCl and 80% hot ethanol for HPLC analysis.

In yet another embodiment of present invention, HPLC analysis was performed with a Shimadzu LC-10A system (Shimadzu corporation, Japan) and the chromatograph was equipped with a 5µm nucleosil C₁₈ column (250 mm long, 4 mm ID, Waters, USA) using Mc Ilvaline buffer (0.1 M ammonium acatate/acetic acid buffer pH 4.7) at a flow rate of 1ml/min and detected with a UV detector (280 nm) and with a fluorescence detector (xenon arc lamp; excitation at 280nm and emission at 314 nm)

In yet another embodiment of present invention, tyrosine at a concentration of 3mg/40ml was fed to the cultures of *Beta vulgaris,* which were at exponential stage of their growth and the effect on Dopa, and dopamine accumulation was recorded.

The following examples are given by way of illustration of the present invention and through should not be considered to limit the scope of invention.

### EXAMPLE 1

Hairy root cultures of *Beta vulgaris* were obtained upon infection of an explant by a wild strain of *Agrobacterium rhizogenes.* 300 mg fresh weight of hairy roots of *Beta vulgaris* (4-day old) was inoculated into 40 ml nutrient medium comprising of Murashige and Skoog's salts in a 150 ml Erlenmeyer's flask. The pH of the medium was adjusted to 5.8 before autoclaving for 15 min at 121°C at 15-lb pressure. The hairy roots were sub-cultured every 2 weeks into fresh MS (Murashige-Skoog) medium with 3% sucrose and without any phytohormone addition and were kept in dark on a rotary shaker (90 rpm). The maximum biomass was recorded on 20^{th} day of culture time as 6.75 g fresh weight (FW) per 40ml culture medium, which further decreased to reach a minimum of 5.2 g FW/40 ml culture medium on 24^{th} day of culture time.

### EXAMPLE 2

300 mg fresh weight of hairy root cultures of *Beta vulgaris* from 4-day old cultures were inoculated into 40 ml nutrient medium comprising of Murashige and Skoog's salts in 150 ml Erlenmeyer's flasks. At time intervals of 6,12,15,20 and 24 days of culture period, the hairy roots were extracted for L-Dopa and dopamine using 80% hot ethanol, 0.1 N HCl and 2% formic acid in a mortar and pestle using neutralized glass powder. The extracts were centrifuged, filtered, concentrated and used for HPLC analysis of L-Dopa and dopamine. Betalaine content in the hairy roots was measured after extracting the roots in acidified water (ascorbic acid -water), centrifuged at 5000X g for 15 minutes. The supernatants were collected and the optical density was measured spectrophotometrically at 480nm and 540 nm for betaxanthins and betacyanins, respectively.

Dopa and dopamine contents were quantified by HPLC analysis of the samples and were compared with those of authentic standards. The identification of the peaks was more prominent when a fluorescence detector was used.

The extractability of L-Dopa and dopamine from *B. vulgaris* using various solvents is shown in table 1.

**Table 1: Extractability of dopa and dopamine from hairy root cultures of Beta vulgaris using different solvents.**

| **80 % Ethanol** | | | **0.1 N HCl** | | **2% Formic acid** | |
|---|---|---|---|---|---|---|
| **Time period** | **Dopa** | **Dopamine** | **Dopa** | **Dopamine** | **Dopa** | **Dopamine** |
| **(days)** | **(µg/g DW)** | **(mg/g DW)** | **(µg/g DW** | **(mg/g DW)** | **(µg/g DW** | **(mg/g DW)** |
| 6 | 17.2 | 0.217 | 20 | 0.375 | *30 | 0.425 |
| 12 | 11 | 0.413 | 15.12 | 0.525 | 21.2 | 0.676 |
| 15 | 5 | 0.512 | 9 | 0.732 | 16 | *0.975 |
| 20 | ND | 0.312 | 2.3 | 0.560 | 7.4 | 0.721 |
| 24 | ND | 0.191 | ND | 0.213 | ND | 0.312 |

Among the solvents used for the extraction of dopa and dopamine, 2% formic acid was proved good compared with 80% hot ethanol and 0.1 N HCl. The Dopa production was analyzed using 2% formic acid as extracting solvent, which was, recorded more content in the initial stages of culture (exponential phase) i.e. 6th day of culture time. The dopamine content was more during 15^{th} day of culture time, which later showed a decrease. When extracted with 2% formic acid, the Dopa extraction levels were 1.7 and 1.5 fold higher, than that of 80% ethanol and 0.1 N HCl extracted ones respectively. Even dopamine recovery was higher when 2% formic acid was used for extraction which is 1.9 and 1.4 fold higher when compared with that of 80% ethanol and 0.1 N HCl extracted ones respectively. Based on the results, further extractions were carried out with 2% formic acid in all experiments with *B. vulgaris* hairy root cultures for production of Dopa and dopamine.

### EXAMPLE 3

300 mg fresh weight of hairy roots of *Beta vulgaris* (4-day old) were inoculated into 40 ml nutrient medium comprising of Murashige and Skoog's salts in a 150 ml Erlenmeyer's flask. The pH of the medium was adjusted to 5.8 before autoclaving for 15 min at 121°C at 15-1b pressure. Tyrosine (dissolved in few drops of 0.1N NaOH the made the volume with distilled water) at a concentration of 3mg/40 ml culture medium was added on the 6-day of culture period. The hairy roots were extracted with 2% formic acid in a mortar and pestle using neutralized glass powder. The extracts were centrifuged, filtered using Whatman #1 filter paper, concentrated and used for HPLC analysis. Dopa content increased from 32 µg/g DW from 6^{th} day of culture time to 46µg/g DW on 15^{th} day, which later decreased to 9µg/g DW on 24^{th} day. The L-Dopa content in this case is 1.53 fold higher when compared to unfed cultures. Dopamine content increased from 0.412 mg/g DW on 6^{th} day to 1.972 mg/g DW on 20^{th} day and later decreased further to reach a value of 0.762mg/g DW on 24^{th} day. Dopamine content in this case (Fed with tyrosine) is 2.02 fold higher when compared to control (Unfed) cultures. Betalaine content also has shown increase compared with control. Maximum betalaine accumulation was recorded on 20^{th} day of culture time as 13.1 mg/g DW from an initial value of 3.1 mg/g DW on 6^{th} day, which again decreased to 8 mg/g DW on 24^{th} day. Thus, this study provides valuable information for the right time (culture time) to harvest the hairy roots for better accumulation of L-Dopa and dopamine and the suitable solvent for their extraction. The results are shown in table 2.

**Table2: Dopa and dopamine contents in tyrosine fed B. vulgaris hairy root cultures**

| Time period | Fresh weight | Dopa | Dopamine | Betalaine |
|---|---|---|---|---|
| (days) | (g/ 40 ml culture vol) | (µg/gDW) | (mg/g DW) | (mg/g DW) |
| 6 | 1.91 | 32 | 0.412 | 3.1 |
| 12 | 4.2 | 44 | 1.067 | 7.2 |
| 15 | 5.4 | 46 | 1.427 | 9 |
| 20 | 7.3 | 22 | 1.972 | 13.1 |
| 24 | 6.1 | 9 | 0.762 | 8 |

**The main advantages of the present investigation are**
1. *In vitro* culture of hairy roots are used for production of Dopa and dopamine
2. As hairy roots exhibit hormonal autotrophy, the process requires a relatively cheaper medium for the phytochemical production
3. The faster growth and genetic as well as biochemical stability of hairy roots is an added advantage
4. The identification of the right stage of culture time for maximum accumulation of Dopa and dopamine would facilitate this process a commercially feasible one.
5. The metabolites Dopa and dopamine can be extracted easily using 2% formic acid, which is a weak acid, compared with HCl and 80%, hot ethanol.
6. Use of precursors like tyrosine, which are easily available for better production of Dopa and dopamine would be useful.
7. As Dopamine is water soluble, its direct use after extraction in water could be an additional advantage.
8. As hairy roots are more amenable for scale-up in bioreactors, addition of tyrosine for maximum accumulation of these phytochemicals at a large scale.

## Claims

1. A process for the production of Dopa and Dopamine from hairy root culture of *Beta vulgaris,* said process comprising steps of:
a) culturing of hairy roots of *Beta vulgaris* using nutrient media, sterilizing in an autoclave;
b) inoculating hairy roots in the range of 0.1-0.5 g fresh wt/40ml culture medium, grown for 2-24 days to obtain higher growth and metabolites;
c) choosing the right stage of culture of hairy roots for maximum Dopa and Dopamine production based on the biosynthetic pathway of betalaines; and
d) extracting the hairy roots with solvent to obtain Dopa and dopamine.

2. The process as claimed in claim 1, wherein in step (a), the nutrient media used comprising MS medium with 2 to 4 % of sucrose as a major carbon source.

3. The process as claimed in claim 1 wherein step (a), sterilization is carried out for 10 to 30 minutes at a temperature in the range of 110 to 130°C.

4. The process as claimed in claim 1, wherein in step (d), the hairy roots are extracted using formic acid in the range of 1-4 % (v/v), 0.1 N HCl and 80% hot ethanol.

5. The process as claimed in claim 1, wherein in step (d), the extracts are centrifuged, filtered and concentrated under vacuum.

6. The process as claimed in claim 1, wherein addition of formic acid in the range of 1-4 % (V/V) for extraction enhances the production of Dopa and dopamine.

7. The process as claimed in claim 1, wherein addition of tyrosine in the range of 1-5 mg/ 40-ml culture medium to the culture of 3- 7 days old, resulting in enhanced yield of Dopa and dopamine.

8. The process as claimed in claim 1, wherein the hairy roots of beta vulgaris are cultured in nutrient liquid medium aseptically and extracted with 1-5 % formic acid at exponential growth.

9. The process as claimed in claim 1, wherein in step (c), the exponential growth phase for maximum accumulation of Dopa in culture ranges between 5- 8 days.

10. The process as claimed in claim 1, wherein in step (c), the exponential growth phase for maximum accumulation of Dopamine in culture ranges between 12- 16 days.

## Patentansprüche

1. Verfahren zur Herstellung von Dopa und Dopamin aus Haarwurzelkulturen von Beta Vulgaris, welches Verfahren die Schritte umfasst, dass man:
a) Haarwurzeln von Beta Vulgaris unter Verwendung eines Nährmediums kultiviert, in einem Autoclaven sterilisiert;
b) Haarwurzeln im Bereich von 0,1 bis 0,5 g Frischgewicht/40ml Kulturmedium inokuliert, gewachsen für 2 bis 24 Tage um ein höheres Wachstum und Metaboliten zu erhalten;
c) das richtige Kulturstadium der Haarwurzeln für eine maximale Dopa- und Dopaminherstellung wählt, bezogen auf den biosynthetischen Pfad der Betalaine; und
d) die Haarwurzeln mit Lösungsmittel extrahiert um Dopa und Dopamin zu erhalten.

2. Verfahren nach Anspruch 1, worin in Schritt a) das verwendete Nährmedium MS Medium umfasst mit 2 bis 4 % Saccharose als Hauptkohlenstoffquelle.

3. Verfahren nach Anspruch 1, wobei in Schritt a) die Sterilisation 10 bis 30 Minuten bei einer Temperatur im Bereich von 110 bis 130°C ausgeführt wird.

4. Verfahren nach Anspruch 1, wobei in Schritt d) die Haarwurzeln unter Verwendung von Ameisensäure im Bereich von 1 bis 4 % (v/v) 0,1 N HCl und 80% heißem Ethanol extrahiert werden.

5. Verfahren nach Anspruch 1, worin in Schritt d) die Extrakte zentrifugiert, gefiltert und unter Vakuum konzentriert werden.

6. Verfahren nach Anspruch 1, wobei die Zugabe von Ameisensäure im Bereich von 1 bis 4 % (V/V) zur Extraktion die Herstellung von Dopa und Dopamin verbessert.

7. Verfahren nach Anspruch 1, wobei die Zugabe von Tyrosin im Bereich von 1 bis 5 mg/ 40 ml Kulturmedium zu 3 bis 7 Tage alten Kultur zu einer verbesserten Ausbeute von Dopa und Dopamin führt.

8. Verfahren nach Anspruch 1, wobei die Haarwurzeln von Beta Vulgaris in einem flüssigen Nährmedium aseptisch kultiviert werden, und mit 1 bis 5 % Ameisensäure bei exponentiellem Wachstum extrahiert werden,

9. Verfahren nach Anspruch 1, wobei in Schritt c) die exponentielle Wachstumsphase zur maximalen Akkumulation von Dopa in Kulturen im Bereich zwischen 5 und 8 Tagen liegt.

10. Verfahren nach Anspruch 1, wobei in Schritt c) die exponentielle Wachstumsphase zur maximalen Akkumulation von Dopamin in Kulturen im Bereich 12 bis 16 Tage liegt.

## Revendications

1. Procédé pour la production de Dopa et de dopamine à partir de culture de racines chevelues de *Beta vulgaris,* ledit procédé comprenant les étapes consistant à :
a) cultiver des racines chevelues de *Beta vulgaris* en utilisant des milieux nutritifs, stériliser dans un autoclave ;
b) inoculer les racines chevelues dans la plage de 0,1 à 0,5 g en poids/40 ml de milieu de culture frais, cultiver pendant 2 à 24 jours pour obtenir une croissance supérieure et davantage de métabolites ;
c) choisir le stade de culture approprié des hairy roots pour une production maximale de Dopa et de dopamine basée sur la voie métabolique de biosynthèse des bétalaïnes ; et
d) extraire les racines chevelues avec un solvant pour obtenir de la Dopa et de la dopamine.

2. Procédé tel que revendiqué dans la revendication 1, dans lequel à l'étape (a), le milieu nutritif utilisé comprend du milieu MS avec 2 à 4% de saccharose comme source de carbone principale.

3. Procédé tel que revendiqué dans la revendication 1 dans lequel à l'étape (a), la stérilisation est effectuée pendant 10 à 30 minutes à une température dans la plage de 110 à 130°C.

4. Procédé tel que revendiqué dans la revendication 1, dans lequel à l'étape (d), les racines chevelues sont extraites en utilisant de l'acide formique dans la plage de 1 à 4% (v/v), HCl à 0,1 N et 80% d'éthanol chaud.

5. Procédé tel que revendiqué dans la revendication 1, dans lequel à l'étape (d), les extraits sont centrifugés, filtrés et concentrés sous vide.

6. Procédé tel que revendiqué à l'étape 1, dans lequel l'addition d'acide formique dans la plage de 1 à 4% (VN) pour l'extraction augmente la production de Dopa et de dopamine.

7. Procédé tel que revendiqué dans la revendication 1, dans lequel l'addition de tyrosine dans la plage de 1 à 5 mg/40 ml de milieu de culture de 3 à 7 jours, a pour résultat un rendement amélioré en Dopa et en dopamine.

8. Procédé tel que revendiqué dans la revendication 1, dans lequel les racines chevelues de *Beta vulgaris* sont cultivées dans du milieu nutritif liquide aseptiquement et sont extraites avec 1 à 5% d'acide formique en croissance exponentielle.

9. Procédé tel que revendiqué dans la revendication 1, dans lequel à l'étape (c), la phase de croissance exponentielle pour l'accumulation maximale de Dopa dans la culture est comprise entre 5 et 8 jours.

10. Procédé tel que revendiqué dans la revendication 1, dans lequel dans l'étape (c), la phase de croissance exponentielle pour l'accumulation maximale de dopamine en culture est comprise entre 12 et 16 jours.
